# EUROPEAN PATENT SPECIFICATION

(11) **EP 1 898 315 B1**
(45) Date of publication and mention of the grant of the patent: **20.04.2011**
(21) Application number: 07013603.1
(22) Date of filing: 11.07.2007
(51) Int. Cl.: G06F 13/00, H04L 29/00

(54) **Operation system control apparatus, operation system control method and operation system**
Vorrichtung und Verfahren zur Betriebssystemsteuerung sowie Betriebssystem
Appareil de contrôle de système de fonctionnement, procédé de contrôle de système de fonctionnement et système de fonctionnement

(30) Priority: 18.07.2006 JP 2006196069
(43) Date of publication of application: 12.03.2008
(73) Proprietor: OLYMPUS MEDICAL SYSTEMS CORP., Tokyo 151-0072 (JP)
(72) Inventor: Yamaki, Masahide, Tokyo 151-0072 (JP)
(74) Representative: von Hellfeld, Axel

(56) References cited:
- EP-A- 1 193 924
- US-A1- 2003 040 835

## Description

### BACKGROUND OF THE INVENTION

### 1. Field of the Invention

The present invention relates to an operation system control apparatus, an operation system control method and an operation system, and more particularly to an operation system control apparatus, an operation system control method, and an operation system for controlling medical devices through a plurality of communication transmission pathways.

### 2. Description of the Related Art

Recently the endoscope apparatus has been employed for performing the surgical operation. The surgical operation with the endoscope is performed using operation equipment such as the penumoperitoneum device for expanding the abdominal cavity and a high-frequency electrocautery unit for excising or coagulating the body tissue as a treatment device for the operation procedure such that the operator is allowed to perform various medical treatments while observing the view derived from the endoscope.

The endoscope operation system including the aforementioned plural instruments is further provided with a display panel, for example, the liquid crystal panel as display means which allows the operator in the sterilized area to confirm the set state of various instruments, a remote operation device, for example, the remote controller as remote operation means which allows the operator in the sterilized area to operate to change the functions or set values of the various instruments, a center operation panel formed by providing operation switches for various instruments on the tough panel, which allows the medical assistant such as the nursing staff to change the functions or set values of the various instruments in the non-sterilized area in response to the command of the operator, and a microphone which allows the operator to operate the various instruments through voice for easy operation and control of a plurality of instruments, and improvement in the system operability.

The medical device used in the endoscope operation system includes the electric cautery, the ultrasonic device, pneumoperiotoneum device and the like in addition to the electronic endoscope system. The aforementioned instruments are integrally controlled as the system by the operation device arranged under the control of the system controller as disclosed in, for example, Japanese Unexamined Patent Application Publication No. 2003-76786 or No. 2003-70746.

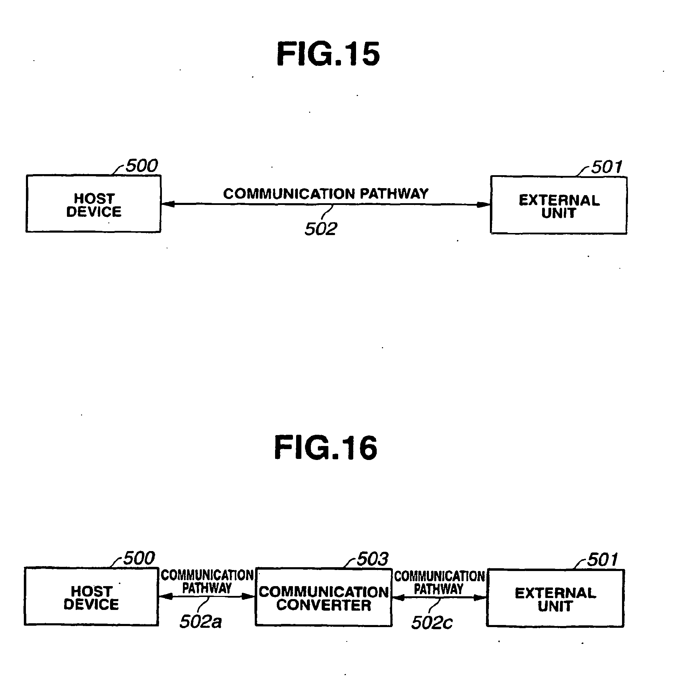

Referring to Fig. 15, the system controller generally controls the aforementioned plurality of medical devices (electronic endoscope system, electric cautery, ultrasonic device, pneumoperitoneum device) through communication thereamong. In this case, the time-out period in the communication set in the host as the system controller is different from the one set in the device as the medical device. Japanese Unexamined Patent Application Publication No. 2000-276824 discloses the control system in which the host identifies the device to set the time-out period for the identified device.

The time-out period in the communication varies depending of the device. However, the time-out period may vary depending on the communication transmission pathway, for example, in the case where the communication converter is provided between the host and the device as shown in Fig. 16. Recently, the devices are required to be arranged in various layouts relative to the host for the purpose of establishing the optimum system. As the communication converter has been provided between the host and the device in accordance with the transmission distance and the transmission capacity, the communication transmission pathway has to be considered with respect to the time-out period for the purpose of establishing the high speed and stable communication control.

The host is required to set the time-out period in accordance with the CPU processing load in a plurality of subsequently released medical devices, and the configuration of the software. The thus set plurality of time-out periods are further required to be held between the host and the plurality of devices.

In the case where the response to the command transmitted to the host and the plurality of devices delays, the transmission/reception error in the device occurs to cause the communication failure. Specifically, various time-out periods set in the host and the devices are monitored to secure quality of the physical layer, protocol (intermediate) layer, and the application layer of the communication interface.

If the layout of the devices is not fixed, the communication load or the required time-out periods may dynamically vary. In the case where the same medical instrument is used, for example, the instrument ID (the category such as the electric cautery and pneumoperitoneum device) is the same, if the instrument used for the operation procedure is dynamically changed or the position of the nursing staff arranged based on the operator's preference becomes different, the layout of the instruments may be dynamically changed accordingly. The entire load of the communication pathway or the response of the specific instrument may vary depending on the layout of the devices to be connected. This may cause the communication failure.

Various types of conversion adapters, hubs and extenders may be used on the communication pathway between the host and the device in the operation environment. As the communication delay time may vary depending on the layout, the time-out period which has been set upon the release of the device may cause the communication failure.

### SUMMARY OF THE INVENTION

Accordingly, it is an object of the present invention to provide an operation system control apparatus and an operation system capable of controlling a plurality of medical devices in optimum and stable manner through plural types of communication transmission pathways.

This problem is solved by an operation system control apparatus according to claim 1, an operation system control method according to claim 3 and an operation system according to claim 5.

An operation system control apparatus according to the present invention is provided with conversion type identification means adapted to identify a type of a signal converter provided on a signal transmission pathway through which a signal is transmitted to and received from a medical device, time-out value storage means adapted to store a time-out value of signal processing on the signal transmission pathway based on the type of the signal converter, and transmission and reception time control means adapted to control a time for transmitting and receiving a signal to and from the medical device based on a minimum time-out value of the signal processing on the signal transmission pathway in accordance with the type of the signal converter stored in the time-out value storage means.

Other features and advantages of the present invention will be apparent by the following description.

### BRIEF DESCRIPTION OF THE DRAWINGS

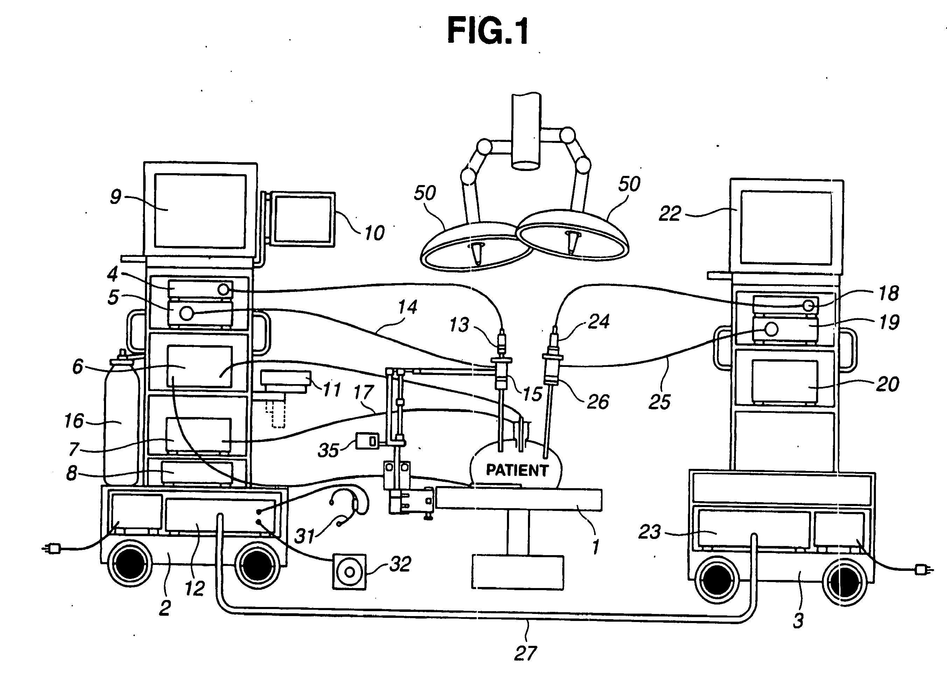

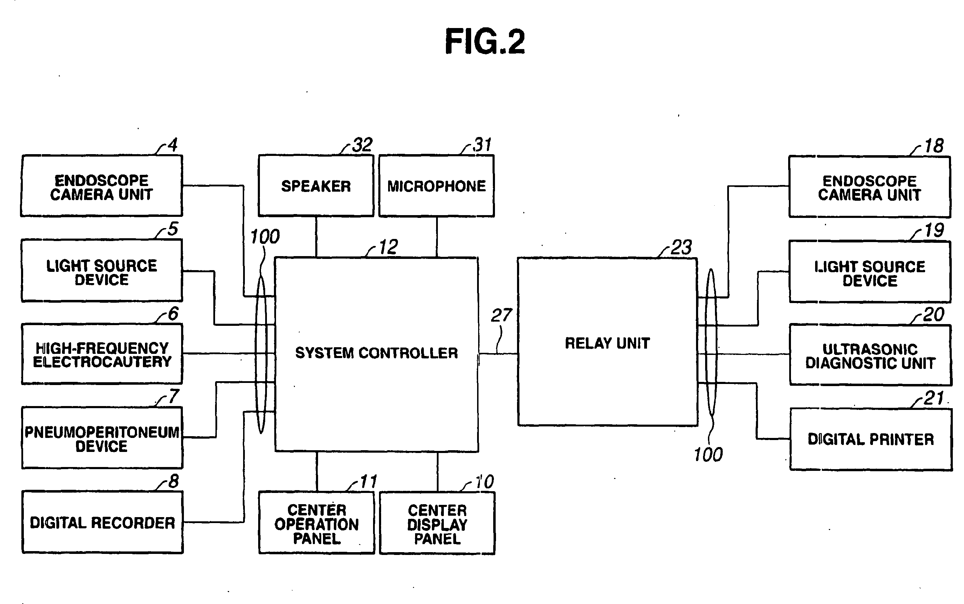

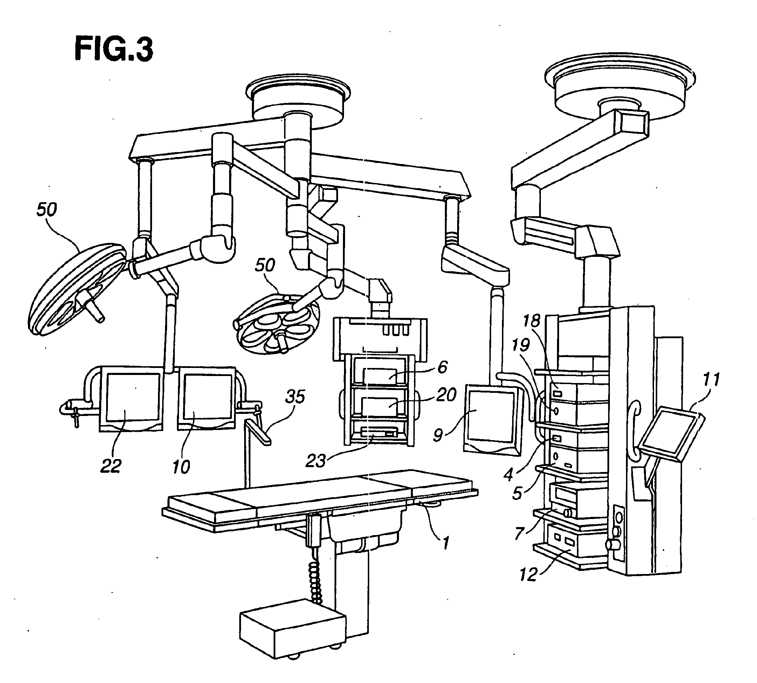

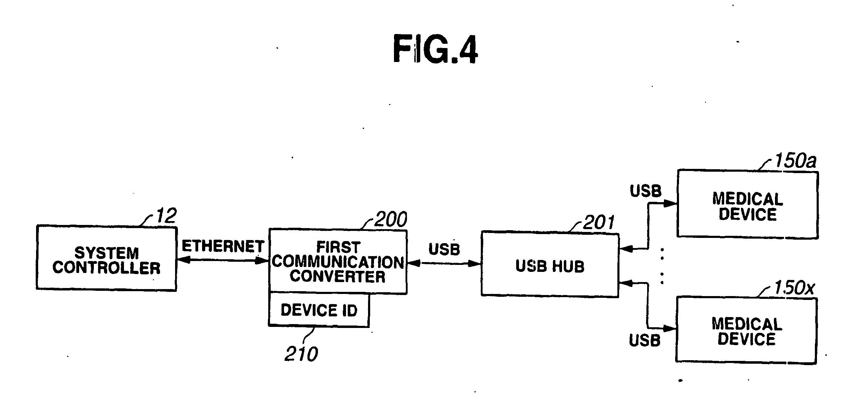

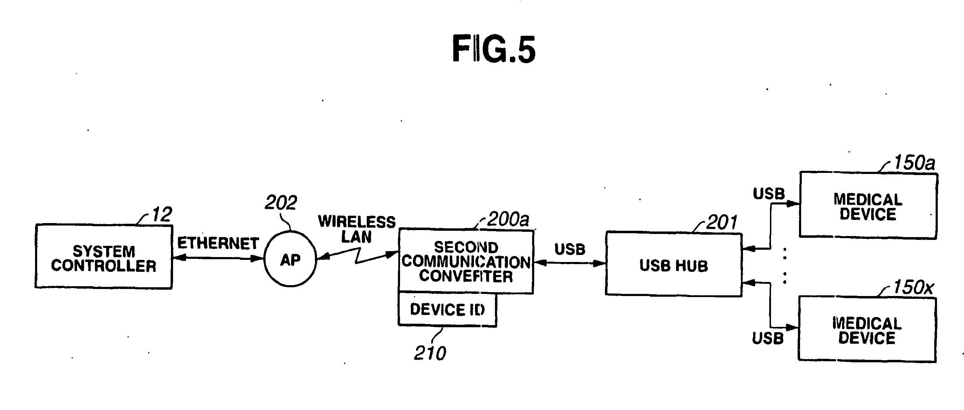

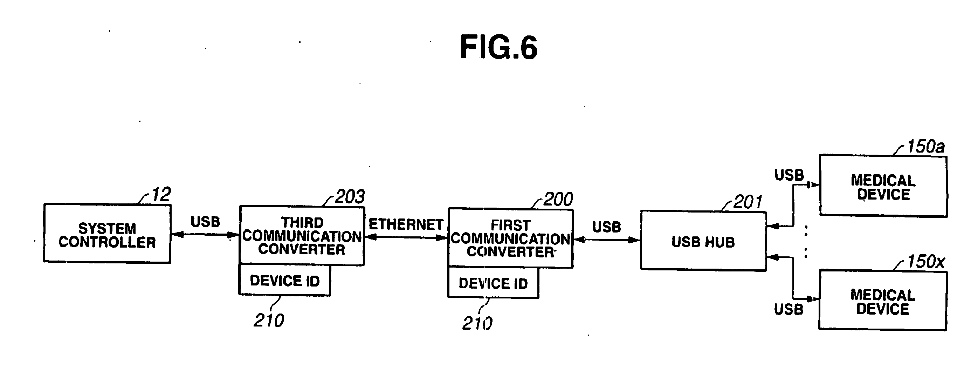

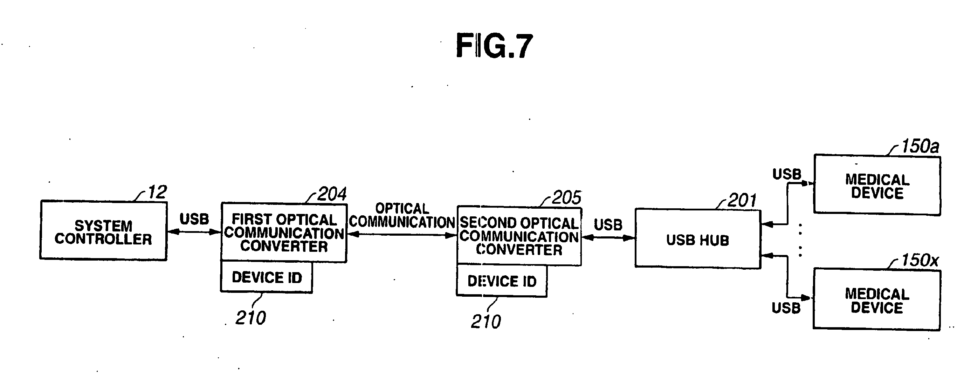

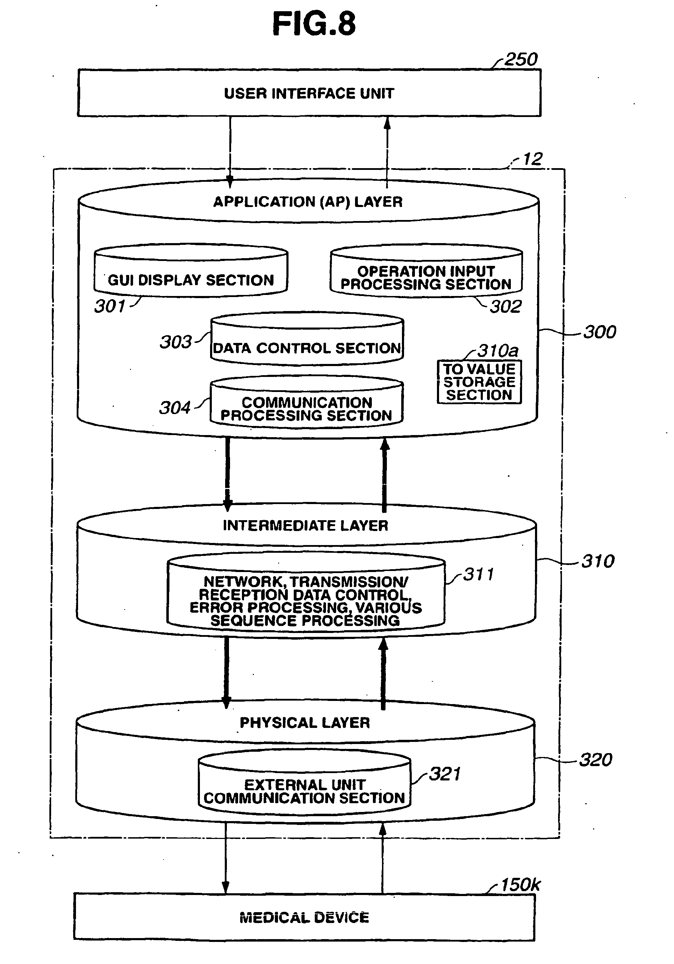

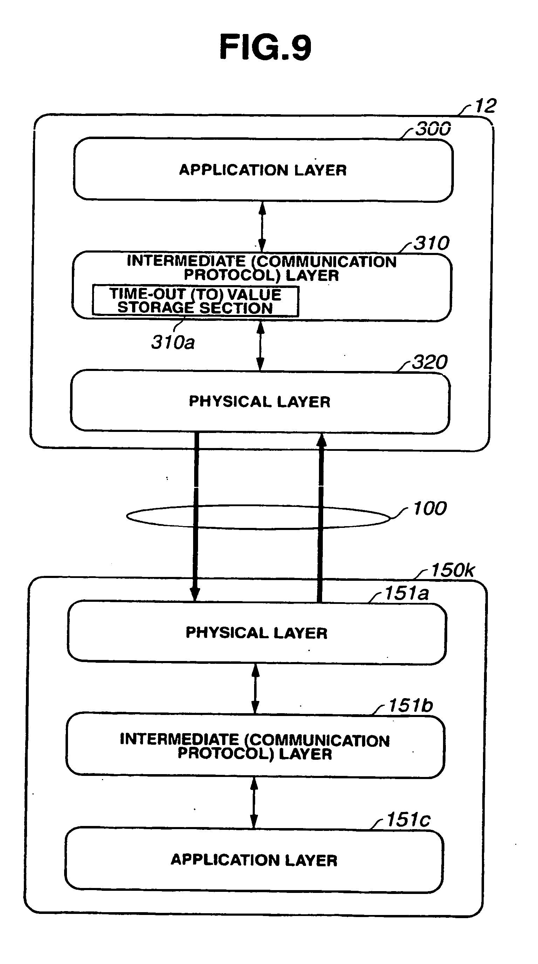

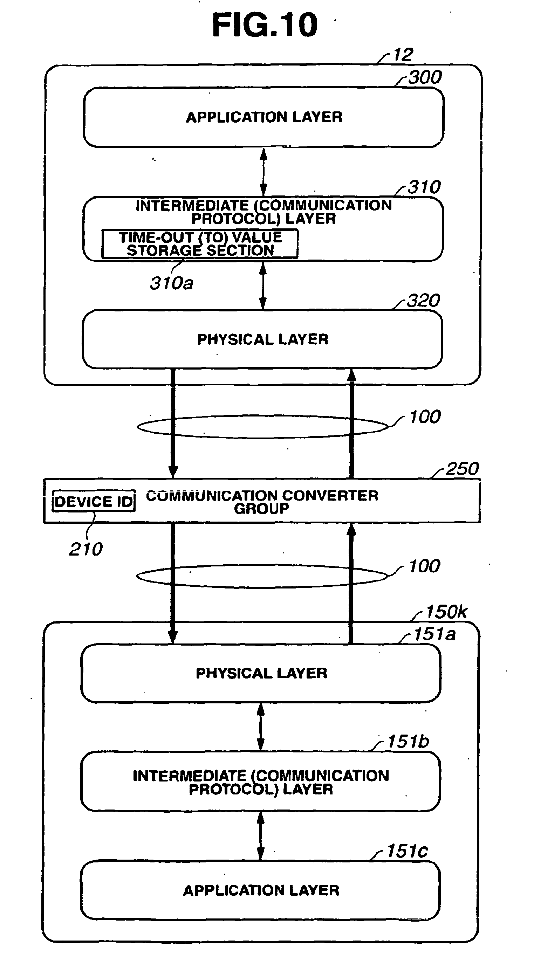

Figs. 1 to 14 are views which relate to a first embodiment of the present invention. Fig. 1 is a view showing an entire structure of the endoscope surgical operation system. Fig. 2 is a block diagram showing the connection among various devices of the endoscope surgical operation system shown in Fig. 1. Fig. 3 is an outline view of a modified example of the layout of the endoscope surgical operation system shown in Fig. 1. Fig. 4 is a first view representing the signal transmission pathway in the endoscope surgical operation system shown in Fig. 1. Fig. 5 is a second view representing the signal transmission pathway in the endoscope surgical operation system shown in Fig. 1. Fig. 6 is a third view representing the signal transmission pathway in the endoscope surgical operation system shown in Fig. 1. Fig. 7 is a fourth view representing the signal transmission pathway in the endoscope surgical operation system shown in Fig. 1. Fig. 8 is a view showing the structure of the system controller shown in Fig. 2. Fig. 9 is a first view showing the connection between the system controller shown in Fig. 8 and the medical device. Fig. 10 is a second view showing the connection between the system controller shown in Fig. 8 and the medical device.

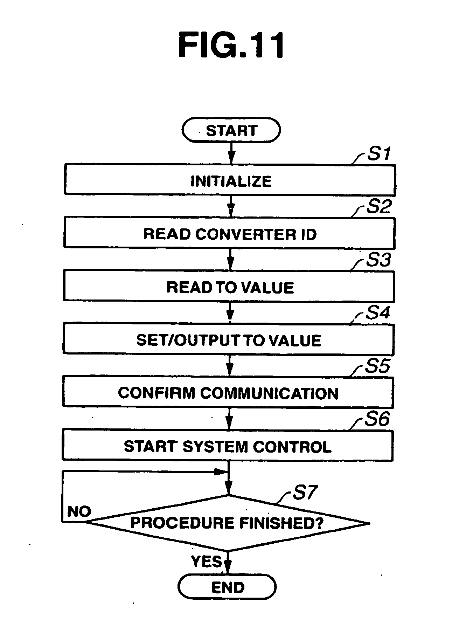

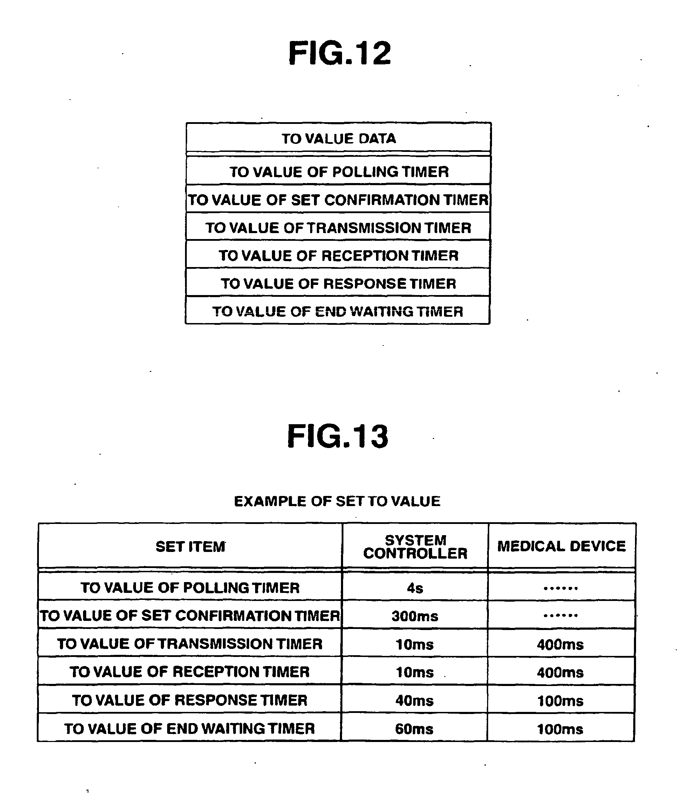

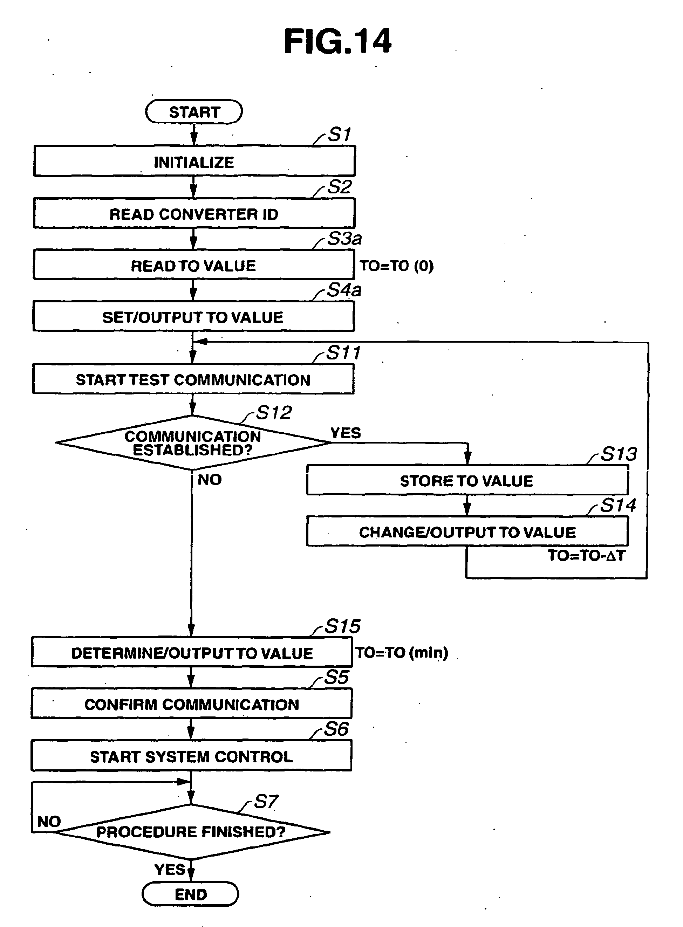

Fig. 11 is a flowchart showing the process executed by the system controller shown in Fig. 8. Fig. 12 is a first view representing the process shown in Fig. 11. Fig. 13 is a second view representing the process shown in Fig. 11. Fig. 14 is a flowchart representing the process as the modified example of the one shown in Fig. 11.

Fig. 15 is a first view showing the related art. Fig. 16 is a second view showing the related art.

### DETAILED DESCRIPTION OF THE PREFERRED EMBODIMENTS

Referring to Figs. 1 and 2, in an endoscope surgical operation system as the operation system according to the embodiment, the peripheral units of the endoscope as a plurality of devices to be controlled are mounted on a first cart 2 and a second cart 3 each placed at the side of a surgical bed 1 on which the patient lies down. Surgical lights 50 are hung from the ceiling above the surgical bed 1 so as to appropriately illuminate on hands of the operator.

An endoscope camera unit 4, a light source device 5, a high-frequency electrocautery (electric cautery) 6, a pneumoperitoneum device 7, a digital video recorder (DVR) 8, a first monitor 9, a center display panel 10, a center operation panel 11 which is remotely operated by the nursing staff or the like, and a system controller 12 for controlling the aforementioned medical devices as the operation system controller are mounted on the first cart 2.

The respective medical devices are connected to the system controller 12 via a communication transmission pathway 100 formed of a communication interface cable such that the bi-directional communication is realized.

An endoscope camera head 13 is connected to the endoscope camera unit 4, and a light guide 14 is connected to the light source device 5. The endoscope camera head 13 and the light guide 14 are connected to an endoscope 15, respectively. A CO₂ tank 16 is connected to the pneumoperitoneum device 7. A pneumoperitoneum tube 17 extending from the pneumoperitoneum device 7 to the patient serves to supply CO₂ gas into the abdominal cavity of the patient.

Meanwhile, an endoscope camera unit 18, a light source device 19, an ultrasonic diagnostic unit 20, a second monitor 22 and a relay unit 23 are mounted on the second cart 3. The respective peripheral units of the endoscope are connected to the relay unit 23 via the unshown communication transmission pathway 100 formed of the communication interface cable such that the bi-directional communication is realized.

An endoscope camera head 24 is connected to the endoscope camera unit 18, and a light guide 25 is connected to the light source device 19, respectively. The endoscope camera head 24 and the light guide 25 are connected to an endoscope 26.

The system controller 12 and the relay unit 23 are connected via a system interface cable 27 such that the bi-directional communication is realized.

The center operation panel 11 is formed of a display unit such as a liquid crystal display and a touch sensor integrally formed on the display unit.

The center operation panel 11 includes a display function which displays each state of the respective devices, and the operation switch as the setting screen, for example, and an operation function performed through the operation switch by touching a predetermined area of the touch sensor.

The center operation panel 11 allows the touch panel (TP) function including the display function and the operation function to perform the same operation as the direct operation of the peripheral devices of the endoscope through the system controller 12.

The system controller 12 is connectable to the microphone 31 for inputting the voice. The system controller 12 allows the voice recognition section (not shown) to recognize the voice inputted through the microphone 31. The system controller 12 allows the voice generation section (not shown) to output the voice guidance corresponding to the recognized voice through a speaker 32. Execution of the aforementioned process ensures the system controller 12 to control the respective devices through the voice guidance.

The microphone 31 is used as the remote operation means for centrally performing the remote operation of the peripheral devices frequently used by the operator, and allows the operator in the sterilized area to fully perform setting and operation of the frequently used peripheral devices of the endoscope through the voice.

A remote controller 35 is disposed around the surgical bed 1, which allows the operator to remotely operate the frequently used peripheral devices.

The respective medical devices may be mounted not only on the first cart 2 and the second cart 3 but also on the ceiling suspension system with higher mobility which is suspended from the ceiling as shown in Fig. 3. The endoscope surgical operation system according to the embodiment may be freely arranged to allow various layouts.

As the endoscope surgical operation system according to the embodiment is structured to allow the free layout, the system controller 12 may be connected to the respective medical devices 150a to 150x via various communication transmission pathways as shown in Figs. 4 to 7.

The medical devices 150a to 150x denote the endoscope camera unit 4, the light source device 5, the high-frequency electrocautery (electric cautery) 6, the pneumoperitoneum device 7, the digital video recorder (DVR) 8, the first monitor 9 and the center display panel 10 which are mounted on the first cart 2, and the endoscope camera unit 18, the light source device 19, the ultrasonic diagnostic unit 20, the second monitor 22 and the relay unit 23 which are mounted on the second cart 3 as shown in Fig. 1.

More specifically, referring to Fig. 4, the system controller 12 transmits/receives a protocol signal of Ethernet™ to/from a first communication converter 200 via an Ethernet™ port. The first communication converter 200 is a protocol converter which converts the protocol signal of Ethernet™ into the USB protocol signal, and includes a device ID section 210 for self-identification. The first communication converter 200 transmits/receives the USB protocol signal to/from the plurality of medical devices 150a to 150x via a plurality of USB hubs 201. As the transmission distance of the protocol signal of Ethernet™ is approximately 100 m which is substantially long, the aforementioned structure allows the medical devices to be arranged relatively far from the system controller 12.

Referring to Fig. 5, the system controller 12 transmits/receives the protocol signal of EthernetTM to/from an access point (AP) 202 via the Ethernet™ port. The access point (AP) 202 converts the protocol signal of Ethernet™ into a wireless LAN signal so as to be wirelessly transmitted to/received from a second communication converter 200a. The second communication converter 200a is a protocol converter which converts the wireless LAN signal into the USB protocol signal, and includes the device ID section 210 for self-identification. The further communication transmission pathway is the same as the one shown in Fig. 4. The aforementioned structure allows wireless signal transmission/reception between the system controller 12 and the medical devices. Accordingly, more flexible arrangement of the medical devices may be realized without requiring the cable.

Referring to Fig. 6, the system controller 12 transmits/receives the USB protocol signal to/from a third communication converter 203 via a USB port. The third communication converter 203 is a protocol converter which converts the USB protocol signal into the Ethernet™ protocol signal to be transmitted to/received from the first communication converter 200, and includes the device ID section 210 for self-identification. The further communication transmission pathway is the same as the one shown in Fig. 4. The aforementioned structure allows the signal transmission/reception between the system controller 12 and the medical devices via the USB port.

Referring to Fig. 7, the system controller 12 transmits/receives the USB protocol signal to/from a first optical communication converter 204 via the USB port. The first optical communication converter 204 is a protocol converter which converts the USB protocol signal into an optical signal to be transmitted to/received from a second optical communication converter 205, and includes a device ID section 210 for self-identification. The second optical communication converter 205 is a protocol converter which converts the optical signal into the USB protocol signal, and includes the device ID section 210 for self-identification. The further communication transmission pathway is the same as the one shown in Fig. 4. The aforementioned structure allows transmission/reception of the information through the optical signal between the system controller 12 and the medical devices as well as the high speed/high capacity communication.

Referring to Fig. 8, the hardware/software system of the system controller 12 has a layered structure including an application layer 300, an intermediate layer 310, and a physical layer 320 and the like. The application layer 300 includes a GUI display section 301 and an operation input processing section 302 for controlling the center display panel 10, the center operation panel 11, the remote controller 35 and the like as the user interface equipment used by the user. The application layer 300 further includes a data control section 303 as the conversion type identification means for controlling the entire system, and a communication processing section 304 as the transmission/reception time control means which allows the communication with the intermediate layer 310. The communication processing section 304 communicates with the intermediate layer 310 and the physical layer 320 based on the time-out value (TO value) stored in the TO value storage section 310a as the time-out value storage means.

The intermediate layer 310 includes an intermediate processing section 311 for executing networking, transmission/reception data control, error processing, and various sequence processing. The intermediate processing section 311 controls the communication between the intermediate layer 310 and the physical layer 320 such that control of data transmission to/reception from the application layer 300 is appropriately executed.

The physical layer 320 includes an external device communication section 321 as a physical communication section which transmits/receives data to/from the medical device 150k (k = a to x) via the corresponding port in accordance with the predetermined physical protocol.

Referring to Fig. 9, the hardware/software system of each of the medical devices 150k (k=a to x) is also formed of a layered structure including an application layer 151c, an intermediate layer 151 b, and a physical layer 151a. In the simple connection mode, data are transmitted/received between the physical layer 320 of the system controller 12 and the physical layer 151a of the medical device 150k (k = a to x) via the communication interface cable 100.

Meanwhile, referring to Fig. 10, the communication converter group 250 (communication converters 200, 200a, 203 to 205, and the access point 202 as shown in Figs. 4 to 7) is arranged between the physical layer 320 of the system controller 12 and the physical layer 151a of the medical device 150k (k = a to x) for the purpose of allowing the flexible layout of the respective medical devices. The data are transmitted/received between the physical layer 320 of the system controller 12 and the physical layer 151 a of the medical device 150k (k = a to x) through the communication converter group 250 via the communication interface cable 100.

In the embodiment, based on the ID information of the device ID section 210 of the communication converter group 250, the communication converter is identified. The time-out period is adjusted/set on the signal transmission pathway through the communication converter group 250 at each signal transmission pathway including the communication converter. The communication between the system controller 12 and the medical device 150k (k = a to x) is, thus, established based on the optimum time-out period.

The time-out period is adjusted/set not only as the period between the physical layer 320 of the system controller 12 and the physical layer 151a of the medical device 150k (k = a to x) but also as the period among the application layer, the intermediate layer and the physical layer of the respective devices.

The function of the thus structured embodiment will be described referring to the flowchart shown in Fig. 11 and views of Figs. 12 and 13.

Referring to Fig. 11, upon start of the operation procedure, in step S1, the system controller 12 executes initialization of the system to confirm the existence of the communication converter group 250 on the signal transmission pathway.

The system controller 12 reads the ID information of the device ID section 210 of the communication converter group 250 confirmed in step S2 by the data control section 303 as the conversion type identification means such that the device type of the communication converter group 250 is identified.

In accordance with the device type of the communication converter group 250 identified by the communication processing section 304 as the transmission/reception time control means in step S3, the system controller 12 reads the time-out value (TO value) data for the respective processing which are stored in the TO value storage section 310a as shown in Fig. 12.

Data of the time-out (TO) values for the respective procsesings stored in the TO value storage section 310a are stored as the table data for the system controller 12 and the respective medical devices 150k as shown in Fig. 13. The system controller 12 sets its own time-out value depending on the device type of the communication converter group 250 by the communication processing section 304 serving as the transmission/reception time control means in step S4. The time-out value of the medical device 150k is outputted to the corresponding medical device 150k from the system controller 12 via the communication transmission pathway on which the communication converter group 250 is provided.

The values shown in the table of Fig. 13 are exemplified as being optimum time-out values stored for the communication converter group 250 and the medical device 150k, respectively.

The system controller 12 establishes the communication with the medical device 150k connected via the communication transmission pathway on which the communication converter group 250 is provided using data of the time-out (TO) values for the respective processings set by the communication processing section 304 as the transmission/reception time control means in step S5.

Thereafter, the system controller 12 starts controlling the system in step S6, and continues the system control in step S7 until the end of the operation procedure is confirmed.

In the embodiment, the system controller 12 identifies the device type of the communication converter group 250 on the signal transmission pathway. Depending on the identified device type of the communication converter group 250, the system controller 12 sets the time-out values for the system controller 12 and the medical device 150k connected via the communication transmission pathway on which the communication converter group 250 is provided such that the communication is established. The system controller 12 allows execution of the optimum and stable communication even if the system controller 12 is connected to the medical device 150k via various communication transmission pathways.

In the embodiment, the system controller 12 sets data of the time-out (TO) values for the respective processings which are stored in the TO value storage section 310a to the system controller 12 and the medical device 150k. However, the system controller 12 is allowed to execute the process as shown in Fig. 14.

More specifically, referring to Fig. 14, after execution of steps S1 to S4, the system controller 12 executes the test communication between the system controller 12 and the medical device 150k based on the data of the time-out (TO) values for the respective processings set in step S 11.

The system controller 12 determines whether the communication with the medical device 150k has been established in step S12. The data of the time-out (TO) values of the respective processings set in steps S 1 to S4 have sufficient margins. In this case, the communication between the system controller 12 and the medical device 150k is ensured owing to sufficient time-out value (length of time).

When it is determined that the communication with the medical device 150k has been established, the system controller 12 stores the time-out value (TO value = TO) obtained at this time in the TO value storage section 310a in step S 13. Subsequently, the system controller 12 decrements the TO value by a predetermined value ΔT in step S 14. The process then returns to step S11.

After the repetitive execution of the cycle from steps S 11 to S 14, when it is determined that the communication between the system controller 12 and the medical device 150k has failed in step S12, the system controller 12 determines the minimum time-out value (TO value = T(min)) to allow establishment of the communication, which has been lastly stored in the TO value storage section 310a in step S 13, and sets the data to the system controller 12 and the medical device 150k so as to execute the process from steps S5 to S7.

Execution of the process shown in Fig. 14 allows the system controller 12 to set the optimum and shorter time-out value in addition to the effect obtained in the process shown in Fig. 11, thus realizing more optimum, more stabilized and higher communication.

In the embodiment, the basic communication quality and response may be realized by the layout for the cable communication such that the operability and the stability of the communication between the host (system controller 12) and the device (medical device 150k) may be established.

In the medical environment which dynamically changes the layout for the subsequent use of each case, the display updating timing of the communication, the data response waiting time and the like may be flexibly adjusted in accordance with the quality policy of the operator or hospital.

The function is expandable to the cable layout (shown in Fig. 4), the extension layout (shown in Figs. 6 and 7), and the wireless layout (shown in Fig. 5) depending on the operation procedure or the operator's preference while maintaining the quality of the devices.

## Claims

1. An operation system control apparatus (12) comprising:
conversion type identification means (210) adapted to identify a type of a signal converter (250) provided on a signal transmission pathway (100) through which a signal is transmitted to and received from a medical device (150);
time-out value storage means (310a) adapted to store a time-out value of signal processing on the signal transmission pathway (100) based on the type of the signal converter (250); and
transmission and reception time control means (304) adapted to control a time for transmitting and receiving a signal to and from the medical device (150) based on a minimum time-out value of the signal processing on the signal transmission pathway (100) in accordance with the type of the signal converter (250) stored in the time-out value storage means (310a).

2. The operation system control apparatus (12) according to Claim 1, wherein the signal converter (250) is adapted to convert a first protocol signal into a second protocol signal on the signal transmission pathway (100).

3. An operation system control method comprising:
conversion type identification step for identifying a type of a signal converter (250) provided on a signal transmission pathway (100) on which a signal is transmitted to and received from a medical device (150);
time-out value storage step for storing a time-out value of a signal processing on the signal transmission pathway (100) based on the type of the signal converter (250); and
transmission and reception time control step for controlling a time of transmitting and receiving a signal to and from the medical device (150) based on a minimum time-out value of the signal processing on the signal transmission pathway (100) in accordance with the type of the signal converter (250) stored in the time-out value storage step.

4. The operation system control method according to Claim 3, wherein the signal converter (250) converts a first protocol signal into a second protocol signal on the signal transmission pathway (100).

5. An operation system including a plurality of medical devices used for an operation, an operation system control apparatus according to claim 1 or 2 adapted to control the medical devices (150), and a signal converter (250) provided on a signal transmission pathway (100) on which a signal is transmitted and received between the operation system control apparatus and the medical devices (150).

## Patentansprüche

1. Operationssystem-Steuervorrichtung (12) mit:
- einer Umsetzungstyp-Erkennungseinrichtung (210), die dafür ausgelegt ist, einen Typ eines Signalumsetzers (250) zu erkennen, der in einem Signalübertragungsweg (100) bereitgestellt ist, über den ein Signal an ein medizinisches Gerät (150) gesendet und von diesem empfangen wird,
- einer Time-Out-Wert-Speichereinrichtung (310a), die dafür ausgelegt ist, einen Time-Out-Wert einer Signalverarbeitung auf dem Signalübertragungsweg (100) basierend auf dem Typ des Signalumsetzers (250) zu speichern, und
- einer Sende- und Empfangszeitsteuereinrichtung (304), die dafür ausgelegt ist, eine Zeit zum Senden und Empfangen eines Signals an das und von dem medizinischen Gerät (150) basierend auf einem Mindest-Time-Out-Wert der Signalverarbeitung auf dem Signalübertragungsweg (100) gemäß dem in der Time-Out-Wert-Speichereinrichtung (310a) gespeicherten Typ des Signalumsetzers (250) zu steuern.

2. Operationssystem-Steuervorrichtung (12) nach Anspruch 1,
wobei der Signalumsetzer (250) dafür ausgelegt ist, auf dem Signalübertragungsweg (100) ein erstes Protokollsignal in ein zweites Protokollsignal umzusetzen.

3. Operationssystem-Steuerverfahren mit:
- einem Umsetzungstyp-Erkennungsschritt zum Erkennen eines Typs eines Signalumsetzers (250), der in einem Signalübertragungsweg (100) bereitgestellt ist, über den ein Signal an ein medizinisches Gerät (150) gesendet und von diesem empfangen wird,
- einem Time-Out-Wert-Speicherschritt zum Speichern eines Time-Out-Werts einer Signalverarbeitung auf dem Signalübertragungsweg (100) basierend auf dem Typ des Signalumsetzers (250), und
- einem Sende- und Empfangszeitsteuerschritt zum Steuern einer Zeit zum Senden und Empfangen eines Signals an das und von dem medizinischen Gerät (150) basierend auf einem Mindest-Time-Out-Wert der Signalverarbeitung auf dem Signalübertragungsweg (100) gemäß dem im Time-Out-Wert-Speicherschritt gespeicherten Typ des Signalumsetzers (250).

4. Operationssystem-Steuerverfahren nach Anspruch 3,
wobei der Signalumsetzer (250) auf dem Signalübertragungsweg (100) ein erstes Protokollsignal in ein zweites Protokollsignal umsetzt.

5. Operationssystem, das eine Mehrzahl für eine Operation verwendeter medizinischer Geräte, eine Operationssystem-Steuervorrichtung nach Anspruch 1 oder 2, die dafür ausgelegt ist, die medizinischen Geräte (150) zu steuern, und einen Signalumsetzer (250) umfasst, der in einem Signalübertragungsweg (100) bereitgestellt ist, über den ein Signal zwischen der Operationssystem-Steuervorrichtung und den medizinischen Geräten (150) gesendet und empfangen wird.

## Revendications

1. Appareil de commande de système d'opération (12) comprenant :
un moyen d'identification de type de conversion (210) adapté à identifier un type d'un convertisseur de signaux (250) prévu sur un chemin de transmission de signaux (100) par lequel un signal est transmis à un et reçu d'un dispositif médical (150) ;
un moyen de stockage de valeur de temporisation (310a) adapté à stocker une valeur de temporisation de traitement de signaux sur le chemin de transmission de signaux (100) sur la base du type du convertisseur de signaux (250) ; et
un moyen de commande de durée de transmission et de réception (304) adapté à commander une durée pour transmettre et recevoir un signal à un et d'un dispositif médical (150) sur la base d'une valeur de temporisation minimum du traitement de signaux sur le chemin de transmission de signaux (100) conformément au type du convertisseur de signaux (250) stockée dans le moyen de stockage de valeur de temporisation (310a).

2. Appareil de commande de système d'opération (12) selon la revendication 1, dans lequel le convertisseur de signaux (250) est adapté à convertir un premier signal de protocole en un deuxième signal de protocole sur le chemin de transmission de signaux (100).

3. Procédé de commande de système d'opération comprenant :
une étape d'identification de type de conversion pour identifier un type d'un convertisseur de signaux (250) prévu sur un chemin de transmission de signaux (100) sur lequel un signal est transmis à un et reçu d'un dispositif médical (150) ;
une étape de stockage de valeur de temporisation pour stocker une valeur de temporisation d'un traitement de signaux sur le chemin de transmission de signaux (100) sur la base du type du convertisseur de signaux (250) ; et
une étape de commande de durée de transmission et de réception pour commander une durée pour transmettre et recevoir un signal à un et d'un dispositif médical (150) sur la base d'une valeur de temporisation minimum du traitement de signaux sur le chemin de transmission de signaux (100) conformément au type du convertisseur de signaux (250) stockée à l'étape de stockage de valeur de temporisation.

4. Procédé de commande de système d'opération selon la revendication 3, dans lequel le convertisseur de signaux (250) convertit un premier signal de protocole en un deuxième signal de protocole sur le chemin de transmission de signaux (100).

5. Système d'opération incluant une pluralité de dispositifs médicaux utilisés pour une opération, un appareil de commande de système d'opération selon la revendication 1 ou 2 adapté à commander les dispositifs médicaux (150), et un convertisseur de signaux (250) prévu sur un chemin de transmission de signaux (100) sur lequel un signal est transmis et reçu entre l'appareil de commande de système d'opération et les dispositifs médicaux (150).
